Europäisches Patentamt

European Patent Office

Office européen des brevets

(11) Veröffentlichungsnummer: **0 306 651**
**A1**

## EUROPÄISCHE PATENTANMELDUNG

(21) Anmeldenummer: 88110839.3

(22) Anmeldetag: 07.07.88

(51) Int. Cl.⁴: **C07H 15/04**

(30) Priorität: 05.09.87 DE 3729843

(43) Veröffentlichungstag der Anmeldung:
15.03.89 Patentblatt 89/11

(84) Benannte Vertragsstaaten:
**BE DE ES FR GB IT NL**

(71) Anmelder: **HÜLS AKTIENGESELLSCHAFT**
**Patentabteilung / PB 15 - Postfach 13 20**
**D-4370 Marl 1(DE)**

(72) Erfinder: **Lüders, Harald, Dr.**
**Langeoogstrasse 81**
**D-4350 Recklinghausen(DE)**
Erfinder: **Hofmann, Peter, Dr.**
**Dormagener Strasse 42**
**D-4370 Marl(DE)**

(54) **Verfahren zur Herstellung von Alkyl-oligoglycosiden.**

(57) Alkyloligoglycoside und Alkylglycoside mit $C_8$- bis $C_{24}$-Alkylgruppen, die als Tenside im Haushalt und im Kosmetikbereich von Interesse sind, können durch Glycosidierung und Umglycosidierung hergestellt werden.

Hellfarbige Alkyloligoglycoside und Alkylglycoside werden aus Sacchariden und Alkoholen durch Glycosidierung und Umglycosidierung unter Zusatz von Salzen anorganischer Säuren oder von mehrwertigen gesättigten Carbonsäuren sowie durch Bleichung der Endprodukte mit Peroxiden hergestellt.

Herstellung von hellfarbigen Alkyloligoglycosiden.

EP 0 306 651 A1

## Verfahren zur Herstellung von Alkyloligoglycosiden

Die Erfindung betrifft ein Verfahren, nach dem mit Hilfe von Komplexierungsmitteln hellfarbige Alkyloligoglycoside und Alkylglycoside, die Alkylgruppen mit 8 bis 24 C-Atomen aufweisen, hergestellt werden können.

Alkyloligoglycoside und Alkylglycoside mit $C_8$- bis $C_{24}$-Alkylresten können ganz oder teilweise aus nachwachsenden Rohstoffen hergestellt werden. Diese Alkyloligoglycoside und Alkylglycoside gewinnen wegen ihrer interessanten Tensideigenschaften bei gleichzeitig sehr guter biologischer Abbaubarkeit zunehmend an Bedeutung. Für Anwendungen im Haushalt und im Kosmetikbereich müssen diese Produkte hohen ästhetischen Ansprüchen genügen. Man ist daher an Verfahren interessiert, nach denen Alkyloligoglycoside und Alkylglycoside in transparenten, farblich schönen wäßrigen Lösungen hergestellt werden können.

Zur Herstellung von Alkyloligoglycosiden und Alkylglycosiden mit langkettigen Alkylgruppen stellt man im allgemeinen zunächst durch Glycosidierung von Sacchariden mit kurzkettigen Alkoholen Alkyloligoglycoside und Alkylglycoside mit $C_1$- bis $C_6$-Alkylgruppen her. Diese Produkte werden dann mit langkettigen Alkoholen durch Umglycosidierung bei erhöhter Temperatur in die gewünschten Alkyloligoglycoside und Alkylglycoside übergeführt. Die so hergestellten Produkte sind jedoch dunkel gefärbt.

Nach EP 165 721 kann die Farbe derartiger Produkte durch mehrstufige Bleichung mit Wasserstoffperoxid verbessert und durch Zusatz von Schwefeldioxid freisetzenden Verbindungen stabilisiert werden. Der Aufhellungseffekt ist ohne Schwefeldioxid von nur kurzer Dauer.

Nach EP 77 167 können bei der Umsetzung von Alkoholen mit Aldosen oder Ketosen Reduktionsmittel wie hypophosphorige Säure oder schwefelige Säure zugesetzt werden. Dadurch wird die Farbe der Alkylglycoside ver bessert. Geringe Mengen an Reduktionsmittel sind bei diesem Verfahren jedoch nur dann wirksam, wenn unter Ausschluß von Sauerstoff gearbeitet wird.

Es sind auch vorbeugende Maßnahmen bekannt. So erhält man nach EP 102 558 farblich verbesserte $C_3$- bis $C_5$-Alkylglucoside, wenn man die Glucosidierung in Gegenwart eines Alkalisalzes einer Borsäure durchführt.

Bei der Herstellung von langkettigen Alkylsacchariden können nach US 4 465 828 auch die Hydroxypolycarbonsäuren Citronensäure, Weinsäure und Äpfelsäure zur Farbverbesserung beitragen. Die hier erzielten Verbesserungen der Lichtdurchlässigkeit der Lösungen sind jedoch für viele Anwendungen zu gering.

Nach US 4 483 979 können Farbstoffe aus Alkylpolysacchariden extrahiert werden. Dieses aufwendige Verfahren erfordert wasserfreie, polare Lösemittel. Außerdem werden nach diesem Verfahren Teile der Alkylpolysaccharide mit extrahiert.

Nach EP 99 183 können wasserhaltige Polysaccharide mit Alkoholen zu hellfarbigen Alkylglycosidlösungen umgesetzt werden. Das Verfahren erfordert jedoch Cosolventien wie Methanol und Aceton und führt zu Reaktionsgemischen mit einem hohen Anteil an unumgesetztem Saccharid.

EP 132 064 zeigt, daß Alkylglycosidlösungen nach beendeter Reaktion durch Alkalialkoholate oder Alkalisalze von Carbonsäuren neutralisiert werden können. Natriumethylat führt dabei im Vergleich zu Natriumhydroxid zu einer Absorptionsverminderung im sichtbaren Bereich von nur etwas mehr als 50 %.

Die bekannten Verfahren zur Herstellung von farblich verbesserten Alkyloligoglycosiden sind umständlich, erfordern z. T. teure Chemikalien oder führen noch nicht zu den gewünschten Farbqualitäten.

Aufgabe der vorliegenden Erfindung ist es, ein vereinfachtes und in der Wirkung verbessertes Verfahren zur Herstellung von hellfarbigen Alkyloligoglycosiden und Alkylglycosiden mit $C_8$- bis $C_{24}$-Alkylgruppen bereitzustellen.

Die Aufgabe wird erfindungsgemäß dadurch gelöst, daß man

1. bei der Herstellung der Produkte, während der Glycosidierung und/oder Umglycosidierung, Salze anorganischer Säuren oder mehrwertige, gesättigte Carbonsäuren, deren Salze oder deren Ester als Komplexierungsmittel zusetzt,

2. die Produkte der Glycosidierung oder der Umglycosidierung in alkoholischer Lösung ggf. mit einem Adsorptionsmittel behandelt und

3. nach Destillation der Alkohole die hergestellten Produkte oder ihre wäßrigen Zubereitungen mit Peroxidverbindungen bleicht.

Zur Herstellung der $C_8$- bis $C_{24}$-Alkyloligoglycoside und -Alkylglycoside führt man zuerst eine Glycosidierung durch. Ausgangsverbindungen dafür sind Hexosen, Pentosen, oligomere Saccharide oder deren Gemische. Beispiele dafür sind Glucose, Mannose, Galactose, Sorbose, Fructose, Xylose, Arabinose, Ribose, Lyxose, Lactose und Maltose. Dabei kann man handelsübliche, wasserhaltige Stärkehydrolysatsirupe wie Dextrose- oder Glucosesirupe oder Maltosesirupe verwenden. Die Produkte können auch Oligosaccharide enthalten. Vorzugsweise geht man von Glucosepräparaten wie Dextrose oder Dextrosesirupen aus.

Für die Glycosidierung werden kurzkettige primäre oder sekundäre Alkohole mit 1 bis 6 C-Atomen eingesetzt. Vorzugsweise verwendet man n-Butanol.

Die Produkte der Glycosidierung sind die Ausgangsverbindungen der Umglycosidierung. Es sind $C_1$-bis $C_6$-Alkylglycoside und -Alkyloligoglycoside mit mittleren Oligomerisationsgraden von 1 bis etwa 6. Vorzugsweise sind es Butylglucoside und Butyloligoglucoside.

Bei der Umglycosidierung werden diese Produkte mit $C_8$-bis $C_{24}$-Alkoholen umgesetzt. Beispielsweise können dabei native Tensidalkohole, wie sie unter anderem auch bei der Hydrierung von Fettsäuren oder Fettsäu rederivaten entstehen, vollsynthetische Ziegleralkohole und Oxoalkohole eingesetzt werden. Sie können verzweigte und unverzweigte Alkylgruppen enthalten. Vorzugsweise enthalten die Alkohole 8 bis 20 C-Atome.

Die Umglycosidierung erfolgt im allgemeinen bei 80 bis 140 °C, vorzugsweise bei 90 bis 120 °C. Sie kann diskontinuierlich oder kontinuierlich durchgeführt werden. Sie wird nach einer (mittleren) Reaktionszeit von 0,5 bis 4 Stunden beendet. Glycosidierung und Umglycosidierung werden in insgesamt 1,5 bis 8 Stunden durchgeführt.

Die Produkte der Umglycosidierung sind $C_8$- bis $C_{24}$-Alkyloligoglycoside und -Alkylglycoside. Sie weisen mittlere Oligomerisationsgrade von 1 bis etwa 10 auf. Vorzugsweise werden nach dem erfindungsgemäßen Verfahren $C_8$- bis $C_{24}$-Alkyloligoglucoside und -Alkylglucoside hergestellt.

Als hellfarbig werden die Produkte bezeichnet, deren 50 %ige wäßrige Lösungen eine Jodfarbzahl von unter 30 aufweisen.

Die bei der Glycosidierung und bei der Umglycosidierung verwendeten Alkohole dienen auch als Lösemittel für die Ausgangsverbindungen und die Produkte.

Als Katalysatoren für die Glycosidierung und Umglycosidierung können starke Mineralsäuren, organische Säuren oder auch stark saure Ionenaustauscher eingesetzt werden. Beispielsweise kann man Schwefelsäure oder p-Toluolsulfonsäure verwenden.

Als Komplexierungsmittel werden vorzugsweise Alkalisalze von anorganischen Säuren verwendet. Besonders bevorzugt werden Alkalisalze der Phosphorsäure, Polyphosphorsäure, Fluorwasserstoffsäure und Cyanwasserstoffsäure.

Vorzugsweise werden auch mehrwertige gesättigte Carbonsäuren, deren Alkalisalze oder deren Methyl- oder Ethylester als Komplexierungsmittel verwendet. Diese mehrwertigen Carbonsäuren enthalten mindestens 2, vorzugsweise 2 bis 5, Carboxylgruppen. Beispiele dafür sind die Nitrilotriessigsäure und ihr Triethylester sowie die Ethylendiamintetraessigsäure und ihre Natriumsalze.

Die Komplexierungsmittel werden, bezogen auf eingesetztes Saccharid, in Konzentrationen von 0,001 bis 10 Gew.-%, vorzugsweise von 0,01 bis 1 Gew.-%, zugegeben.

Die Produkte der Glycosidierung und Umglycosidierung können mit Adsorptionsmitteln behandelt werden. Dazu rührt man vorzugsweise 0,01 bis 10 Gew.-% Aktivkohle, bezogen auf die Lösung, bei 10 bis 140 °C ein und filtriert die Aktivkohle am Ende der Behandlung wieder ab.

Nach Destillation der langkettigen Alkohole kann der Rückstand mit Wasser zu einer pumpbaren Lösung verarbeitet werden. Der Rückstand oder eine wäßrige Zubereitung wird mit Peroxidverbindungen wie Wasserstoffperoxid, Peressigsäure, Perbenzoesäure oder Peroxydischwefelsäure bei 50 bis 100 °C, vorzugsweise bei 70 bis 90 °C, gebleicht. Wasserstoffperoxid wird als Bleichmittel bevorzugt und wird vorzugsweise in Konzentrationen von 0,05 bis 5 Gew.-%, bezogen auf die Umglycosidierungsprodukte, angewendet.

Durch das vorliegende Verfahren können hellfarbige $C_8$- bis $C_{24}$-Alkyloligoglycoside und - Alkylglycoside bei geringem apparativen Aufwand und geringem Aufwand an Chemikalien hergestellt werden. Das Verfahren kann diskontinuierlich und kontinuierlich durchgeführt werden. Die Zudosierung von Komplexierungsmitteln ist technisch einfach durchführbar.

Das Verfahren liefert Produkte mit sehr guter Farbqualität, die für viele Anwendungszwecke geeignet sind, bei denen neben den Tensideigenschaften und der biologischen Abbaubarkeit auch ästhetische Gesichtspunkte gefragt sind.

Die Farbverbesserung ist erheblich. Sie ist stärker als durch eine bloße Addition der Maßnahmen zu erwarten war.

Die Komplexierungsmittel werden in so geringen Mengen verwendet, daß sie die Tensideigenschaften der Produkte nicht beeinträchtigen und deshalb in den Produkten verbleiben können. Die Komplexierungsmittel sind im allgemeinen sogar in der Lage, die Waschmitteleigenschaften der Produkte noch zu verbessern.

Das Verfahren zur Herstellung der hellfarbigen Produkte wird allgemein wie folgt durchgeführt:

Man geht von einem Saccharid oder einem Saccharidsirup aus und setzt 0,5 bis 10 Mole kurzkettige

3

Alkohole pro Mol Saccharideinheit, 2 bis 200 Milliäquivalente Säure pro kg Alkohole und außerdem Komplexierungsmittel zu. Die Glycosidierung erfolgt dann bei 80 bis 130 °C unter Destillation von Wasser.

Die erhaltenen Glycosidierungsprodukte können mit Aktivkohle gereinigt werden, bevor man ihnen zur Umglycosidierung 0,5 bis 10 Mole langkettige Alkohole pro Mol Saccharideinheit, 2 bis 200 Milliäquivalente Säure pro kg Alkohol und vorzugsweise erneut Komplexierungsmittel zusetzt. Die Gesamtmenge an Komplexierungsmittel soll im Bereich von 0,001 bis 10 Gew.-%, bezogen auf die eingesetzten Saccharide, liegen. Die Umglycosidierung wird bei 80 bis 140 °C vorzugsweise bei vermindertem Druck unter Destillation der kurzkettigen Alkohole durchgeführt, wobei man oberflächenaktive Alkyloligoglycoside und Alkylglycoside gelöst in langkettigen Alkoholen erhält.

Nach Neutralisation kann eine Aktivkohlebehandlung erfolgen. Man kann auch sofort überschüssige langkettige Alkohole im Vakuum destillieren. Der Rückstand der Destillation wird vorzugsweise in Wasser gelöst und dann mit Wasserstoffperoxid bei etwa 70 °C gebleicht.

Durch Eindampfen erhält man schließlich die hellfarbenen tensidaktiven Produkte.

## Beispiel 1

In einem 250 ml-Kolben mit Rührer, Rückflußkühler und Wasserabscheider werden 50 g Butylglucosid, 87,5 g n-Butanol, 40 g 70 %iger wäßriger Dextrosesirup, 1,6 ml 2 N butanolische Schwefelsäure und 32 mg Trinatriumpolyphosphat unter Rühren und unter Rückfluß und Wasserabscheidung 3 Stunden bei 700 mbar gekocht, wobei die Temperatur im Bereich von 95 bis 105 °C gehalten wird. Danach ist der Fehling-Test auf Aldehyde negativ.

In die Mischung werden erneut 87,5 g n-Butanol, 40 g 70 %iger wäßriger Dextrosesirup, 1,1 ml 2 N butanolische Schwefelsäure und 32 mg Trinatriumphosphat gegeben, worauf weitere 3 Stunden bei 700 mbar und 95 bis 105 °C unter Wasserabscheidung gekocht wird. Der Fehling-Test ist dann negativ.

Die so erhaltenen Glycosidierungsprodukte gelöst in n-Butanol werden mit 2,5 g Aktivkohlepulver versetzt, 30 Minuten bei Raumtemperatur gerührt und dann filtriert.

228 g Filtrat, 400 g Kokosfettalkohol 12/14, 7,3 ml 2 N butanolische Schwefelsäure und 100 mg Trinatriumpolyphosphat werden in einem 1 l-Kolben mit Destillationsbrücke und Vorlage bei 30 mbar und 110 °C gerührt, wobei n-Butanol abdestilliert. Nach 2 Stunden ist die Umglycosidierung beendet.

Das Reaktionsgemisch wird mit 2 N Natronlauge neutralisiert und dann mit 100 mg Natriumhydrogencarbonat gepuffert. Die Mischung wird 1 Stunde bei 80 °C gerührt und anschließend am Rotationsverdampfer im Ölpumpenvakuum bei einer Badtemperatur von 150 °C eingedampft.

Der Rückstand, im wesentlichen ein $C_{12}$- bis $C_{14}$-Alkyloligoglucosid mit einem Oligomerisationsgrad von 1,2, wird mit gleichen Teilen Wasser in eine 50 %ige Lösung übergeführt.

100 g Lösung werden mit 3,3 ml 30 %iger Wasserstoffperoxidlösung 1 Stunde bei 70 °C gebleicht. Die Lösung weist danach eine Jodfarbzahl von 5 auf.

Durch Eindampfen der gebleichten Lösung im Wasserstrahlvakuum erhält man ein nur leicht beige gefärbtes Produkt.

## Beispiele 2 bis 6

Es wird wie in Beispiel 1 verfahren. Bei gleichen Mengen werden jedoch andere Komplexierungsmittel und teilweise andere langkettige Alkohole verwendet. Die Ergebnisse sind in Tabelle 1 zusammengestellt.

Tabelle 1

| Beispiel | Komplexierungsmittel | langkettiger Alkohol | Jodfarbzahl |
|----------|---------------------|---------------------|-------------|
| 2 | Na F | ALFOL$^R$ 1012* | 15 |
| 3 | Na CN | " | 10 |
| 4 | Malonsäure | " | 15-20 |
| 5 | Nitrilotriessigsäure | Kokosfettalkohol 12/14** | 4- 5 |
| 6 | Ethylendiamintetraessigsäure | " | 7-10 |

*) Alkoholmischung aus etwa 85 % n-Decanol, 8,5 % n-Dodecanol und 6,5 %
n-Tetradecanol der Firma Condea, D-2212 Brunsbüttel
**) Alkoholmischung aus etwa 1 % n-Decanol, 72 % n-Dodecanol, 25 % n-Tetradecanol und
1 % n-Hexanol

**Ansprüche**

1. Verfahren zur Herstellung von hellfarbigen $C_8$- bis $C_{24}$-Alkyloligoglycosiden und -Alkylglycosiden aus Sacchariden und Alkoholen durch Glycosidierung und Umglycosidierung bei erhöhter Temperatur unter Zusatz von Komplexierungsmitteln,
dadurch gekennzeichnet,
daß man

1. bis zur Beendigung der Umglycosidierung Salze anorganischer Säuren oder mehrwertige gesättigte Carbonsäuren, deren Salze oder deren Ester zusetzt,

2. die Produkte der Glycosidierung oder der Umglycosidierung in alkoholischer Lösung ggf. mit einem Adsorptionsmittel behandelt und

3. nach Destillation der Alkohole die Produkte der Umglycosidierung oder ihre wäßrigen Zubereitungen mit Peroxidverbindungen bleicht.

2. Verfahren nach Anspruch 1,
dadurch gekennzeichnet,
daß man Alkalisalze anorganischer Säuren zusetzt.

3. Verfahren nach Anspruch 2,
dadurch gekennzeichnet,
daß man Alkalisalze der Phosphorsäure, Polyphosphorsäure, Fluorwasserstoffsäure oder Cyanwasserstoffsäure zusetzt.

4. Verfahren nach Anspruch 1,
dadurch gekennzeichnet,
daß man mehrwertige gesättigte Carbonsäuren, deren Alkalisalze oder deren Methyl- oder Ethylester zusetzt.

5. Verfahren nach Anspruch 1,
dadurch gekennzeichnet,
daß man 0,001 bis 10 Gew.-% Komplexierungsmittel, bezogen auf die eingesetzten Saccharide, zusetzt.

6. Verfahren nach Anspruch 5,
dadurch gekennzeichnet,
daß man 0,01 bis 1 Gew.-% Komplexierungsmittel zusetzt.

7. Verfahren nach Anspruch 1,
dadurch gekennzeichnet,
daß man mit 0,05 bis 5 Gew.-% Wasserstoffperoxid, bezogen auf die Umglycosidierungsprodukte, bleicht.

8. Verfahren nach Anspruch 1,
dadurch gekennzeichnet,
daß man die Adsorptionsmittelbehandlung mit 0,01 bis 10 Gew.-% Aktivkohle, bezogen auf die Lösung, bei 10 bis 140 °C durchführt.

9. Verfahren nach Anspruch 1,
dadurch gekennzeichnet,
daß als Alkyloligoglycoside und Alkylglycoside hellfarbige $C_8$- bis $C_{24}$-Alkyloligoglucoside und -Alkylglucoside hergestellt werden.

10. Verfahren nach Anspruch 1,
dadurch gekennzeichnet,
daß man die Umglycosidierung bei 80 bis 140 °C in 0,5 bis 4 Stunden durchführt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y,D | EP-A-0 102 558 (BASF)<br>* Ansprüche 1-4 *<br>--- | 1 | C 07 H 15/04 |
| Y,D | EP-A-0 077 167 (ROHM&HAAS)<br>* Seite 5, Zeilen 15-20; Ansprüche 1,7 *<br>--- | 1 | |
| Y | GB-A-2 132 202 (PROCTOR&GAMBLE)<br>* Anspruch 1 * & US-A-4 465 828 (Kat. D)<br>--- | 1 | |
| Y | FR-A-1 549 814 (CORN PRODUCTS)<br>* Seite 2, Spalte 2, Zeilen 8-13 *<br>----- | 1 | |

RECHERCHIERTE SACHGEBIETE (Int. Cl.4)

C 07 H 15/00

Der vorliegende Recherchenbericht wurde für alle Patentansprüche erstellt

| Recherchenort | Abschlußdatum der Recherche | Prüfer |
|---|---|---|
| DEN HAAG | 08-11-1988 | BRENNAN J. |